# EUROPÄISCHE PATENTANMELDUNG

(11) **EP 3 100 763 A1**
(43) Veröffentlichungstag der Anmeldung: **07.12.2016**
(21) Anmeldenummer: 16170562.9
(22) Anmeldetag: 20.05.2016
(51) Int. Cl.: A61N 1/04, A61N 1/08, A61N 1/18, A61N 1/05

(54) **IMPLANTIERBARE ELEKTRODE MIT EINER HAFTVERMITTELNDEN OBERFLÄCHENSTRUKTUR**

(30) Priorität: 02.06.2015 DE 102015108670; 02.06.2015 DE 102015108672; 02.06.2015 DE 102015108671
(71) Anmelder: BIOTRONIK SE & Co. KG, 12359 Berlin (DE)
(72) Erfinder: Kolberg, Gernot, 12161 Berlin (DE); Friedrich, Michael, 14532 Kleinmachnow (DE)
(74) Vertreter: Galander, Marcus

(57) **Zusammenfassung**

Die Erfindung betrifft eine Elektrode (20) mit einer haftvermittelnden Oberflächenstruktur (10).

## Beschreibung

Die vorliegende Erfindung betrifft eine implantierbare Elektrode.

Implantierbare Elektroden zum Einsatz im oder am Herzen sind in Verbindung mit implantierbaren Herzschrittmachern entwickelt worden und seit langem in großer Vielgestaltigkeit bekannt. Bei weitem die größte Bedeutung haben hierbei intrakardial verlegte Elektrodenleitungen erlangt, die über einen transvenösen Zugang direkt in das Herz geführt werden. Für diese Elektroden sind verschiedene Arten der Fixierung an der Innenwand des Herzens beziehungsweise im Trabekelwerk der Herzkammer vorgeschlagen und auch praktisch realisiert worden.

Hierunter befinden sich auch verschiedene Arten von Einschraubelektroden, die am distalen Ende eine Fixierschraube tragen. Daneben gibt es auch intrakardiale Elektroden mit Widerhaken- bzw. Finnenanordnung zur atraumatischen Fixierung im Trabekelwerk. Ferner sind auf spezielle Weise gekrümmte und/oder verzweigte Elektrodenleitungen bekannt, bei denen die vorgeprägte Grundform ein zuverlässiges Anliegen an der Herzwand und damit die sichere Übertragung von Stimulationsimpulsen des Schrittmachers an diese gewährleisten soll.

Während für den dauerhaften Einsatz zur Impulsübertragung von fest implantierten Schrittmachern im Wesentlichen nur intrakardiale Elektroden eingesetzt werden, kommen epikardiale Elektroden vor allem zur temporären Stimulation des Herzens bei oder nach operativen Eingriffen zum Einsatz. Weiterhin werden sie in Form großflächiger Flächenelektroden (Patch-Elektroden) in Verbindung mit implantierbaren Defibrillatoren eingesetzt.

Mittlerweile sind kompakte Schrittmacher entwickelt worden, bei denen die elektrisch aktive Fläche der Elektrode direkt am Gehäusekörper sitzt, also keine Elektrodenleitung zum Elektrodenkopf vorhanden ist (auch als kabellose Schrittmacher bezeichnet).

Ein therapeutisches oder diagnostisches Gerät, das an einem bestimmten Ort wirken soll, muss dort fixiert werden, um bei Bewegungen seine Position zu behalten. Das ist häufig unabdingbar, um den therapeutischen Effekt aufrecht halten zu können. Eine Stimulationselektrode spricht zum Beispiel ein vom Arzt sorgfältig ausgewähltes Zellareal im Herzen an. Für dieses Areal werden die elektrischen Parameter für die Therapie eingestellt. Wenn die Elektrode ihre Position ändert, geht mit großer Wahrscheinlichkeit die therapeutische Wirkung verloren, weil einerseits die Parameter nicht für die neue Position geeignet sind oder weil das Areal nicht den therapeutischen Effekt unterstützt. Im ungünstigen Fall kann es sogar zu einer Patientengefährdung kommen, weil eine Stimulation eines falschen Areals zu gefährlichen Effekten führen kann.

Ein anderer Grund, Komponenten im Gefäßsystem oder im oder auf dem Herzen zu fixieren, ist der, die Komponenten an einer sicheren Position zu halten. Andernfalls würden die Komponenten vom Blutstrom mitgespült werden oder durch die Schwerkraft an Orte gelangen, wo sie dem Patienten gefährlich werden können. So können sie Gefäße verstopfen und Embolien, Herzinfarkt oder Gehirnschlag verursachen.

Eine zuverlässige Fixierung der Elektroden am Implantationsort ist demnach für diagnostische und therapeutische Zwecke unverzichtbar. Bei einer Dislokation der Elektroden kann die gewünschte Funktion nicht mehr gewährleistet werden und es können erhebliche Komplikationen auftreten. Der Fixiermechanismus selbst sollte dabei möglichst geringe Auswirkung auf den Organismus haben. Eine rein mechanische Fixierung durch Einnähen, Verankerungsstrukturen oder Klemmelemente kann das betroffene Gewebe nachhaltig und möglicherweise irreparabel verletzen. Haftvermittelnde Klebstoffe können zu Unverträglichkeitsreaktionen führen und damit fixierte Elektroden lassen sich in der Regel nicht mehr verletzungsfrei von dem anhaftenden Gewebe trennen.

Die genannten Lösungen verursachen demnach häufig eine Verletzung der Gewebestrukturen. Diese Verletzung initialisiert die Bindegewebsproliferation, was die Fixierung positiv unterstützt. Ein Nachteil des Bindegewebes ist jedoch die Veränderung von Zellstrukturen. Diese Veränderung kann sich negativ auf den therapeutischen Effekt auswirken, zum Beispiel durch eine Erhöhung der Reizschwelle bei der Stimulation. Das Einnähen der Komponenten ist sehr sicher, aber mit viel Aufwand verbunden. Eine epikarde Elektrode lässt sich eigentlich nur bei offenem Brustkorb annähen. Dahingegen ist das Einschrauben mit einer gewendelten Nadel oder das Abstützen an den Gefäßwänden immer wieder von Dislokationen begleitet.

Die geschilderten Probleme des Standes der Technik lassen sich mit Hilfe der erfindungsgemäßen implantierbaren Elektrode zum Einsatz im oder am Herzen lösen oder zumindest mindern. Die Elektrode zeichnet sich dadurch aus, dass die Elektrode einen Elektrodenkopf mit einer haftvermittelnden Oberflächenstruktur, vorzugsweise Geckostruktur, umfasst.

Die Erfindung nutzt somit eine alternative Möglichkeit der Verbindung unterschiedlicher Oberflächen über das Phänomen der Trockenadhäsivität. Unter Trockenadhäsivität wird vorliegend die Ausbildung von adhäsiven Kräften zwischen Oberflächen ohne haftvermittelnde Stoffe, wie Klebstoffe, verstanden. Solche Haftsysteme sind beispielsweise auch aus der Natur, z.B. bei Gecko- oder Insekten-Beinen, bekannt. Man nimmt an, dass bei solchen Systemen die Haftkräfte auf van-der-Waals-Kräften basieren. Die hafterzeugende Oberfläche weist dazu eine haftvermittelnde Oberflächenstruktur auf, beispielweise eine Vielzahl von borsten- oder haarförmigen Elemente, die zu einer sehr starken Vergrößerung der zur Verfügung stehenden Kontaktfläche führen. Mit der Vergrößerung der Kontaktfläche nimmt in der Folge die Stärke der bei der Kontaktierung ausgebildeten Adhäsionskräfte zu. Der Einsatz derartiger haftvermittelnder Oberflächenstrukturen zur Anbindung an Gewebe wird beispielsweise von Alborz Mahdavi et al., ,A biodegradable and biocompatible gecko-inspired tissue adhesive', PNAS (2008), Vol. 105, No. 7, 2307-231, vorgeschlagen.

Im Bereich der Herzelektroden kann mit Hilfe der haftvermittelnden Oberflächenstruktur eine Verletzung des Gewebes vermieden werden, wie sie bei herkömmlichen Verfahren (Einnähen oder Einschrauben) auftreten und zu einer Schwächung der therapeutisch nutzbaren Gewebsareale führen kann. Das Vermeiden von Verletzungen macht zum Beispiel auch eine minimalinvasive epikardiale Anwendung ungefährlich, weil nicht mehr versehentlich Herzkranzgefäße bei der Fixierung verletzt werden können. Weiterhin ist eine sehr schnelle Fixierung durch leichtes Andrücken der Komponente an die gewünschte Stelle möglich, so dass neue Implantationsmethoden entwickelt werden können. Heute wird bei der Implantation der größte Aufwand in das Fixieren der Komponenten gelegt. Ein Teil des Implantationsdurchmessers muss heute den Fixierungstools zugeschrieben werden. So ist gerade bei der epikardialen Anwendung eine große Öffnung im Brustkorb erforderlich, um die Elektroden Annähen oder Ein-schrauben zu können. Ebenso sind konventionelle intrakardiale Schraubelektroden mit aktiv rückholbarer Schraube technisch aufwendig und benötigen eine stabile und große Innenwendel, um die Torsion übertragen zu können. Auf den aufwendigen und risikobehafteten Mechanismus kann mit der Erfindung verzichtet werden. Schließlich ermöglicht die haftvermittelnde Oberflächenstruktur ein leichtes und planmäßiges erneutes Ablösen des Implantats. Trotz einer guten Fixierung ist eine Umsetzung der zu fixierenden Komponente möglich, bei der sich die Fixierung auch leicht wieder lösen lässt, ohne sich unbeabsichtigt zu lösen.

Die haftvermittelnde Oberflächenstruktur kann beispielsweise zwischen 10 und 1.000.000 Stäbchen pro Quadratmillimeter aufweisen. Das Verhältnis von Durchmesser und Länge der Stäbchen kann zwischen 1:2 und 1:2000 liegen. Der Querschnitt des Stäbchens kann querprofiliert sein, zum Beispiel ganz oder teilweise rund, dreieckig, rechteckig, quadratisch oder innen hohl. Er kann ein T-Profil haben oder den Umrissen einer Mondsichel entsprechen. Dadurch kann eine Vorzugsbiegerichtung des Stäbchens vorgegeben werden. Alternativ oder in Kombination können die Stäbchen vorgebogen oder schräg angebracht sein. Eine einheitliche Biegerichtung der Stäbchen kann verhindern, dass die Stäbchen sich untereinander verheddern. Die Stäbchen können ferner ein Längsprofil haben. So können sie an der Wurzel, wo sie an der zu fixierenden Komponente anliegen verdickt sein und sich zum Ende hin verjüngen.

Die haftvermittelnde Oberflächenstruktur kann aus sich verästelnden Stäbchen bestehen. Das Ende des letzten Zweiges kann wieder verdickt sein. Die größte Ausdehnung der Verdickung entspricht maximal dem 100-fachen des Stäbchendurchmessers, auf dem die Verdickung sitzt. Das Ende des letzten Zweiges kann auch planar oder abgerundet sein oder spitz auslaufen. Am Ende des letzten Zweiges kann sich eine lappenartige Struktur, ähnlich einer Kelle, befinden, die einseitig angebunden ist. Die lappenartige Struktur ist vorzugsweise einseitig an die Stäbchen angebunden in der Art, dass der Winkel der Stäbchen fortgesetzt wird. So wird bei einer Querkraft der Komponente in die ablösende Richtung (zum Beispiel entgegen dem Uhrzeigersinn) die lappenartige Struktur vom Gewebe geschält, was das Ablösen erheblich erleichtert, während bei Querkraft in die andere Richtung nur eine Scherkraft verursacht wird, die die Fixierung nicht nur nicht ablöst, sondern unterstützt.

Durch Gestaltung der Biegerichtung der Stäbchen auf der Fläche können die Fixations- und Ablösekräfte eingestellt werden. Die Strukturen fixieren besonders gut, wenn möglichst viele Stäbchen gleichzeitig die Zugkräfte aufnehmen. Soll die Fixierung gelöst werden, müssen die Stäbchen möglichst einzeln belastet werden, um auch mit geringen Kräften eine Ablösung zu ermöglichen. Durch die Vorzugsbiegerichtung der Stäbchen kann eine seitlich auf die Komponente wirkende Kraft je nach Richtung in eine Zugkraft oder in eine Druckkraft umgesetzt werden. Eine Kraft entgegen der Stäbchenausrichtung führt zu einer das Stäbchen stauchenden Kraft, die ein Umbiegen des Stäbchens verursacht, in dessen Folge an der fixierenden Oberfläche ein Abrollen stattfindet, das die fixierende Fläche abschält. Dieser Effekt kann auch über mehrere Stäbchenebenen ausgenutzt werden. So kann zum Beispiel nur das untere Ende der Stäbchen mit Vorzugsrichtung ausgestattet sein. Die darauf folgenden zum Bespiel verästelten Strukturen werden abgeschält. Ein gleichwertiger Effekt wird erzielt, wenn die Stäbchen nicht eine Vorzugsbiegerichtung haben, sondern bereits schräg angebracht oder vorgekrümmt sind.

Eine spezielle Ausführung der vorgebogenen oder mit Vorzugsbiegerichtung versehenen Stäbchen ist die, bei der die Stäbchen um einen Drehpunkt, vorzugsweise den Punkt der elektrisch aktiven beziehungsweise sensiblen Fläche in eine Richtung, vorzugsweise entgegen dem Uhrzeigersinn vorgebogen sind. Ein Drehen an der Komponente entgegen dem Uhrzeigersinn rollt jedes einzelne Stäbchenende um die Fixierstelle und schält es ab. So kann die Fixierung durch Andrücken der Komponente oder durch Drehen im Uhrzeigersinn erfolgen. Ein Ablösen erfolgt durch Drehung entgegen dem Uhrzeigersinn.

Neben der genannten tangentialen Ausrichtung der Stäbchen sind weitere Strukturierungen vorstellbar, zum Beispiel eine Fläche, deren Stäbchen in eine Richtung zeigen, ist durch eine Kraft in diese Richtung ablösbar und in die andere Richtung stabil.

Soll die Komponente mit einer orthogonal wirkenden Kraft abgelöst werden, ist es sinnvoll, dass die fixierende Fläche als Membran ausgelegt wird, und die Stäbchen zum Membranmittelpunkt zeigen. Bei senkrechtem Abheben der Komponente lösen sich die Stäbchen von außen nach innen ab. Dieser Vorgang kann alternativ durch einen Stößel, der von innen auf die Membran drückt, oder mit Fluiddruck ausgelöst werden.

Die haftvermittelnde Oberflächenstruktur kann grundsätzlich aus jedem Material gefertigt werden, das sich mit den weiteren Bestandteilen der Elektrode verbinden lässt und das ausreichend verträglich ist für einen intrakorporalen Einsatz. Die haftvermittelnden Oberflächenstrukturen bestehen bevorzugt aus einem polymeren Werkstoff, insbesondere einem Silikon. Weitere mögliche Werkstoffe für die Strukturen umfassen Kohlenstoffmaterialien, insbesondere in Form von Fasern und Nanotubes, Polypropylen, Polytetrafluorethylen (PTFE), Ethylen-Tetrafluorethylen (ETFE), Polycarbonat, Polystyrol, Polylactide, wie zum Beispiel PDLLA, künstliche Spinnenseide, Polyurethane und Copolymere davon, Polyimid, Polyamid, Polyetheretherketon (PEEK), Polysulfon, Polyethylen, Polyoxymethylen (POM), Polyetherblockamid, Chitin, Kollagen, Zellulose, Keratin, Metalle, Glas und Keramik. Die haftungsvermittelnden Oberflächenstrukturen können insbesondere aus einem elektrisch leitfähig Material bestehen, um neben dem mechanisch stabilen Kontakt auch einen elektrischen Kontakt zu ermöglichen. Die Struktur kann mit einer geeigneten Substanz beschichtet werden, wie z. B. Poly(dopaminmethacrylat-co-2-methoxyethylacrylat) (p(DMA-co-MEA)), um die Haftfestigkeit in flüssigen Medien zu verbessern, oder mit Steroiden, um Entzündungsprozesse zu unterdrücken. Weiterhin können Substanzen Einsatz finden, die das Einwachsverhalten fördern.

Eine haftvermittelnde Oberflächenstruktur kann mit unterschiedlichen Verfahren hergestellt werden. Beispielsweise können durch lithographische Verfahren, wie z. B. Elektronenstrahllithographie und Laserlithographie, oder durch Ätzverfahren negative Formen erzeugt werden. In einem anschließenden Gussverfahren wird dann ausgehend von der negativen Form die positive Oberfläche mit haarähnlichen Fortsätzen erzeugt (siehe beispielsweise A.K. Geim et al., Nature Mater. 2, 461-463 (2003) und H. Lee, B.P. Lee and P.B. Messersmith, Nature 448, 338-341 (2007)).

Vorzugsweise ist die haftvermittelnde Oberflächenstruktur auf einer Stirnseite des Elektrodenkopfes der Elektrode angeordnet. Hierdurch keine eine Dislokation der elektrisch aktiven Flächen, die zur Stimulation des angrenzenden Gewebes oder Detektion elektrophysiologischer Vorgänge dienen, wirkungsvoll verhindert werden. Der Elektrodenkopf wird einfach an der vorgesehenen Position an das Gewebe angedrückt und die haftungsvermittelnde Oberflächenstruktur hält den Kopf in der gewünschten Position.

Eine weitere, bevorzugte Ausgestaltung der vorhergehenden Ausführungsform sieht vor, dass der Elektrodenkopf sich ausgehend von einem distalen Ende der Elektrodenleitung tellerförmig aufweitet und der tellerförmig ausgeweitete Bereich des Elektrodenkopfes in Richtung der Elektrodenleitung reversibel umklappbar ausgelegt ist. Der Elektrodenkopf besitzt also eine Art umlaufende Lamelle, die sich in proximale Richtung der Elektrodenleitung umklappen lässt und sich anschließend wieder in die ursprüngliche Lage verlegen lässt oder von alleine zurückstellt. Dies lässt sich beispielweise dadurch erreichen, dass zumindest der den Querschnitt der Elektrodenleitung überkragende Teil des Elektrodenkopfes aus einem Material mit elastischen Eigenschaften, zum Beispiel einem Polymer, geformt ist. Durch die spezielle Formgebung des Elektrodenkopfes kann die haftvermittelnde Oberflächenstruktur und damit potentielle Kontaktfläche zum angrenzenden Gewebe vergrößert werden. Während der minimalinvasiven Implantation liegen die tellerförmig erweiterten Bereiche des Elektrodenkopfes jedoch an der Elektrodenleitung an und werden dort zum Beispiel in einer geeigneten Hülse gehalten, so der Querschnitt hinreichend gering bleibt. Erst am Implantationsort wird die Kopfelektrode wieder aufgeweitet, in dem beispielsweise die erwähnte Hülse zurückgezogen wird. Die Verwendung eines elastischen Materials ermöglicht zusätzlich eine bessere Anpassung der haftungsvermittelnden Oberflächenstruktur an das Gewebe, was die Adhäsionskräfte weiter erhöht.

In Fortbildung der vorgenannten Ausführungsform sind auf der Stirnseite des Elektrodenkopfes Abstandshalter angeordnet, die die haftvermittelnde Oberflächenstruktur überragen. Auf diese Weise kann verhindert werden, dass die haftvermittelnde Oberflächenstruktur mit der Innenseite einer Hülse in Berührung kommt, die den umgeklappten, tellerförmig aufgeweiteten Bereich des Elektrodenkopf während des Führens an den Implantationsort in Position hält. Die Abstandhalter sind also so dimensioniert, das die auf der Stirnseite befindlichen haftvermittelnden Oberflächenstrukturen keine Haftung zur Innenseite der Hülse entwickeln können.

Alternativ kann die haftvermittelnde Oberflächenstruktur so ausgelegt sein, dass eine vom Mittelpunkt des Elektrodenkopfes radial nach außen wirkende Kraft einer Adhäsion der Oberflächenstruktur an einer anliegenden Fläche entgegenwirkt. Mit anderen Worten, die haftvermittelnde Oberflächenstruktur kann so gestaltet werden, dass ein Gleiten entlang der Innenseite der vorgenannten Hülse in distale Richtung möglich ist. Dies lässt sich beispielsweise derart bewerkstelligen, dass die Struktur eine Vielzahl von Stäbchen aufweist, deren Enden zur Stirnseite der Kopfelektrode so abgewinkelt sind, dass sie sich zur Mitte der Stirnseite neigen. Weitere Möglichen der Gestaltung der Biegerichtung der Stäbchen sind bereits vorangehend beschrieben wurden.

Wenn der Elektrodenkopf mittig eine Fixierschraube aufweist, kann nach dem gleichen Prinzip ein allmähliches Herausdrehen der Schraube in Folge der ständigen Gewebsbewegung verhindert werden. Die haftvermittelnde Oberflächenstruktur ist dann so ausgelegt, dass eine mit der Gewinderichtung der Fixierschraube wirkende Kraft einer Adhäsion der Oberflächenstruktur an einer anliegenden Fläche entgegenwirkt. Mit anderen Worten, die Schraube lässt sich ungehindert bis in die gewünschte Tiefe eindrehen, weil die Haftung zwischen der haftvermittelnden Oberflächenstruktur und dem anliegendem Gewebe durch die besondere Formgebung der Struktur immer wieder gelöst wird. Einer Drehung in Gegenrichtung steht jedoch die volle Adhäsionskraft der Struktur entgegen.

Die geschilderten Probleme des Standes der Technik lassen sich ferner mit Hilfe der erfindungsgemäßen implantierbaren Elektrode und mit einer langgestreckten Elektrodenleitung und einem distal an dieser angeordneten Elektrodenkopf lösen oder zumindest mindern. Die Elektrode zeichnet sich dadurch aus, dass die Elektrodenleitung eine haftvermittelnde Oberflächenstruktur, vorzugsweise Geckostruktur, aufweist. Die Strukturen befinden sich also seitlich auf der Elektrodenleitung und ermöglichen eine Fixierung in Gefäßen, zum Beispiel den Coronarvenen, oder an der Herzwandung (endo- oder epikardial).

Eine weitere, Ausführungsform der vorgenannten Elektrode sieht vor, dass die haftvermittelnde Oberflächenstruktur im Bereich einer Vorformung der Elektrodenleitung, die zur Abstützung an einer Gefäßwand dient, angeordnet ist. Die Positionierung von auf spezielle Weise gekrümmten und/oder verzweigten Elektrodenleitungen wird also dadurch unterstützt, dass die vorgeprägte Grundform in vorgegebenen Bereichen eine haftvermittelnde Oberflächenstruktur aufweist. Hierdurch ist ein besonders zuverlässiges Anliegen an der Herzwand gewährleistet.

Die implantierbare Elektrode ist vorzugsweise eine Herzelektrode, zum Beispiel einen (gegebenenfalls auch kabelfreien) Schrittmacher oder Defibrillator. Die Elektrode ist jedoch nicht zwingend auf dieses Anwendungsgebiet beschränkt und kann zum Beispiel ebenso Verwendung in Implantaten zur diagnostischen oder therapeutischen Behandlung der Harnröhre, der Blase, in Mund, Nase oder Speiseröhre, im Verdauungssystem, im Gehörgang oder Uterus finden.

Weitere bevorzugte Ausgestaltungen der Erfindung ergeben sich aus den Unteransprüchen und der nachfolgenden Beschreibung.

Die Erfindung wird nachfolgend anhand eines Ausführungsbeispiels und dazugehöriger Zeichnungen erläutert. Es zeigen:
- Fig. 1: Eine schematische Schnittansicht durch eine haftvermittelnde Oberflächenstruktur einer erfindungsgemäßen Elektrode, die eine Vielzahl von Stäbchen aufweist.
- Fig. 2: Beispielhafte Querschnitte von Stäbchen der haftvermittelnden Oberflächenstruktur.
- Fig. 3: Eine Illustration der Gestaltungsmöglichkeiten der Stäbchen der haftvermittelnden Struktur durch Verzweigung der Stäbchen und Formgebung im Bereich der Enden.
- Fig. 4: Eine schematische Darstellung einer Vorzugsbiegerichtung der Stäbchen einer haftvermittelnden Oberfläche, die ein Fixieren bei Drehung mit dem Uhrzeigersinn beziehungsweise Lösen bei Drehung gegen den Uhrzeigersinn ermöglicht.
- Fig. 5: Eine schematische Darstellung einer Vorzugsbiegerichtung der Stäbchen einer haftvermittelnden Oberfläche, die ein Lösen durch orthogonal wirkende Kräfte ermöglicht.
- Fig. 6: Eine schematische Darstellung einer Vorzugsbiegerichtung der Stäbchen einer haftvermittelnden Oberfläche, die ein Verschieben in eine Richtung ermöglicht.
- Fig. 7: Eine Herzelektrode mit Fixierschraube und einer haftvermittelnden Oberfläche.
- Fig. 8: Eine epikardiale Herzelektrode mit einer haftvermittelnden Oberfläche.
- Fig. 9A bis 9C: Eine weitere Ausführungsform einer Herzelektrode mit Fixierschraube und einer haftvermittelnden Oberfläche in drei verschiedenen Ansichten.
- Fig. 10A und 10B: Zwei weitere Ausführungsformen von Herzelektroden mit einer haftvermittelnden Oberfläche.
- Fig. 11: Eine Elektrodenleitung mit einer haftvermittelnden Oberfläche in einer ersten Ausführungsform.
- Fig. 12: Eine Elektrodenleitung mit einer haftvermittelnden Oberfläche in einer zweiten Ausführungsform.

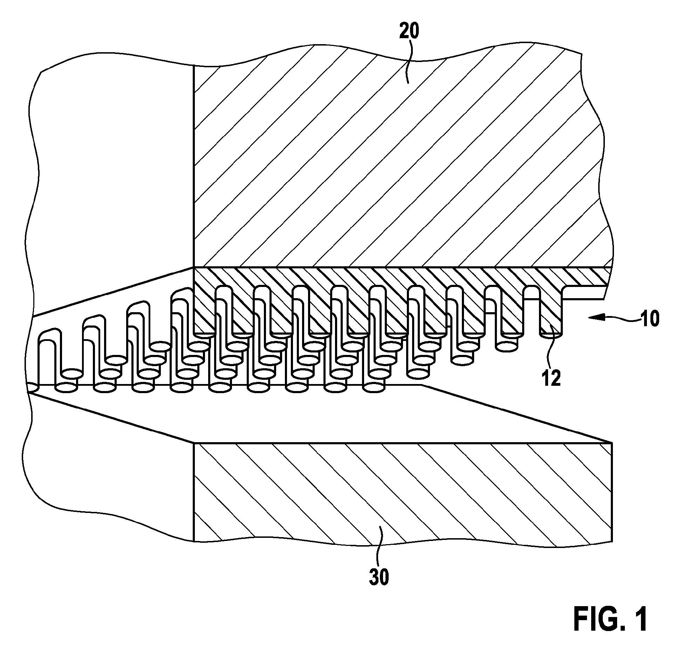

Figur 1 zeigt eine schematische Schnittansicht durch eine haftvermittelnde Oberflächenstruktur 10, die auf einer Oberseite einer Elektrode 20 angeordnet ist. Die Struktur 10 weist eine Vielzahl von Stäbchen 12 auf, die sich nahezu senkrecht von der Oberseite der Elektrode 20 abheben. Die mit der Struktur 10 modifizierte Fläche befindet sich über einer Gewebefläche 30, auf die sie kurz angedrückt werden muss. Die Struktur 10 besteht vorzugsweise aus einem elastischen Material, zum Beispiel Silikon. Bei Andrücken geben die Stäbchen 12 nach, damit die Fläche noch dichter an eine Oberfläche eines Gewebes 30 herangeführt werden kann. Auf diese Weise kann eine Kontaktfläche zwischen den Stäbchen 12 und dem Gewebe 30 und damit Adhäsionskraft zwischen den Komponenten vergrößert werden. Bei Nachlassen des Andruckes halten die Stäbchen 12 den Kontakt zur Oberfläche des Gewebes 30. Mögliche Unebenheiten werden demnach durch die elastische Dehnung der Stäbchen 12 ausgeglichen.

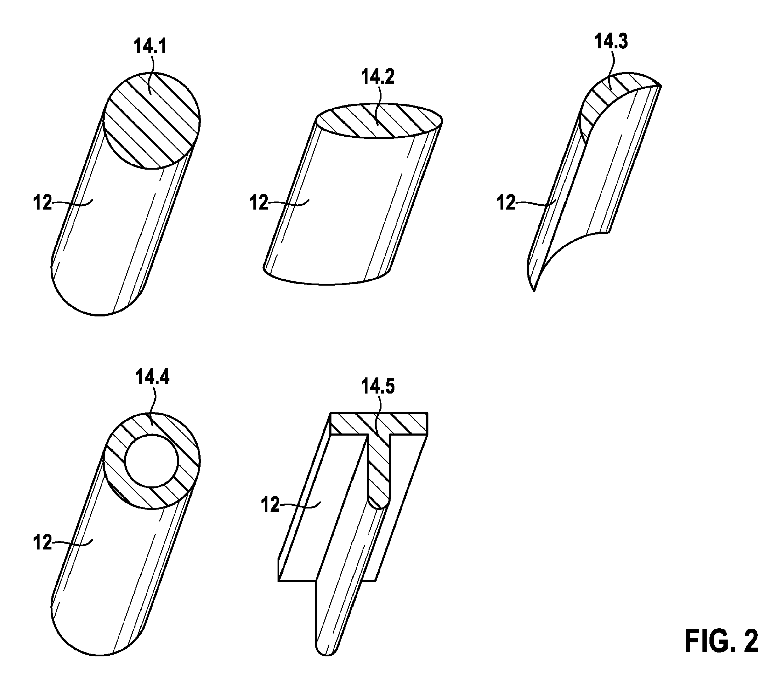

Die Stäbchen 12 können in Längsrichtung einen unterschiedlichen Querschnitt haben. Figur 2 zeigt exemplarisch fünf verschiedene Querschnitte von Stäbchen 12 und deren Einfluss auf das Biegeverhalten. Ein runder Querschnitt 14.1 bedingt keine Biegevorzugsrichtung, ist jedoch herstellungstechnisch besonders einfach realisierbar. Es kann jedoch zum Beispiel erreicht werden, dass ein Stäbchen 12 sich bei Belastung nur in einer Ebene biegt (flacher Querschnitt 14.2) oder dass das Stäbchen 12 sich zusätzlich zu nur einer Ebene in eine Richtung leichter biegt als in die andere Richtung (Sichelquerschnitt 14.3). Ein Stäbchen 14 kann ebenso einen Hohlraum haben (Querschnitt 14.4) oder ein T-Profil aufweisen (Querschnitt 14.5). Damit lässt ich sowohl das Biegeverhalten verändern, als auch die Dehn- und Stauchbarkeit. So hat ein rohrförmiges Stäbchen gegenüber einem voll ausgefüllten eine flachere Federkennlinie. Das ist sinnvoll, weil dadurch größere Höhenunterschiede zwischen Komponente und Gewebe ausgeglichen werden können. Ziel ist, dass alle Stäbchen 12 möglichst die gleiche Kraft von der Komponente zum Gewebe 30 übertragen. Bei einer steilen Federkennlinie würde ein Stäbchen 12, das einen weiten Weg ausgleichen muss, infolge der Dehnung mehr Kraft übertragen und damit schneller wieder abreißen.

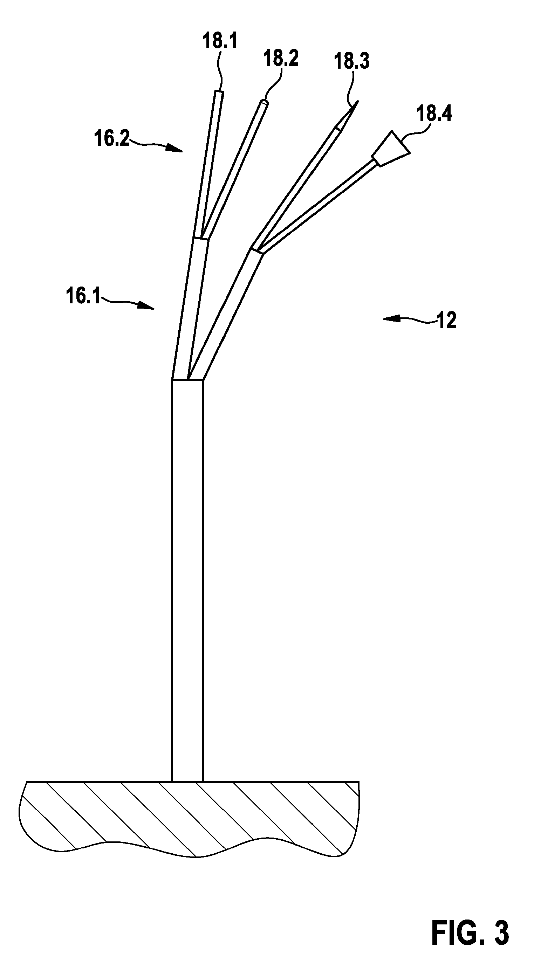

Figur 3 illustriert rein schematisch Ansatzpunkte für die konkrete Optimierung der Form der Stäbchen 12 der haftvermittelnden Struktur in der jeweiligen Applikation. Über solche Stäbchenstrukturen können die Kräfte zwischen Gewebe 30 und Komponente eingestellt werden. So kann das Stäbchen 12 Verzweigungen aufweisen, hier exemplarisch zwei zusätzliche Verzweigungsebenen 16.1 und 16.2. So können zum Beispiel die primären Stäbchenabschnitte steif und lang sein und damit grobe Höhenunterschiede ausgleichen, die Stäbchenabschnitte der ersten Verzweigungsebene 16.1 zum Ausgleich mittlere Höhenunterschiede dienen, während sich die Stäbchenabschnitte der zweiten Verzweigungsebene 16.2 an die Gewebeoberfläche herantasten. Dabei werden die Stäbchenabschnitte vorzugsweise von Ebene zu Ebene kürzer und zierlicher.
Auch die Formgebung im Bereich der Enden der Stäbchen 12 lässt sich variieren. Die Ende kann zum Beispiel gerade abgeschnitten sein (Spitze 18.1), abgerundet werden (Spitze 18.2), spitz auslaufen (Spitze 18.3) oder lappenartig ausgelegt werden (Spitze 18.4). Eine besonders bevorzugte Ausführung ist die einseitig angebundene, lappenartige Struktur (Spitze 18.4). Vorteil dieser Ausführung ist, dass die einseitige Anbindung das Ablösen erleichtert, weil so ein eine Zugkraft in eine Schälbelastung überführt werden kann. Die hohe Adhäsionskraft entsteht aus der Summe der mikroskopischen Fixationsflächen. Entsprechend muss eine ablösende Kraft sehr hoch sein. Wenn die Kraft jedoch in eine Schälbelastung überführt wird, werden die anhaftenden Strukturen Stück für Stück so stark belastet, dass es zur Ablösung kommt. Die lappenartige Struktur kann vom Gewebe 30 wieder abgerollt werden.

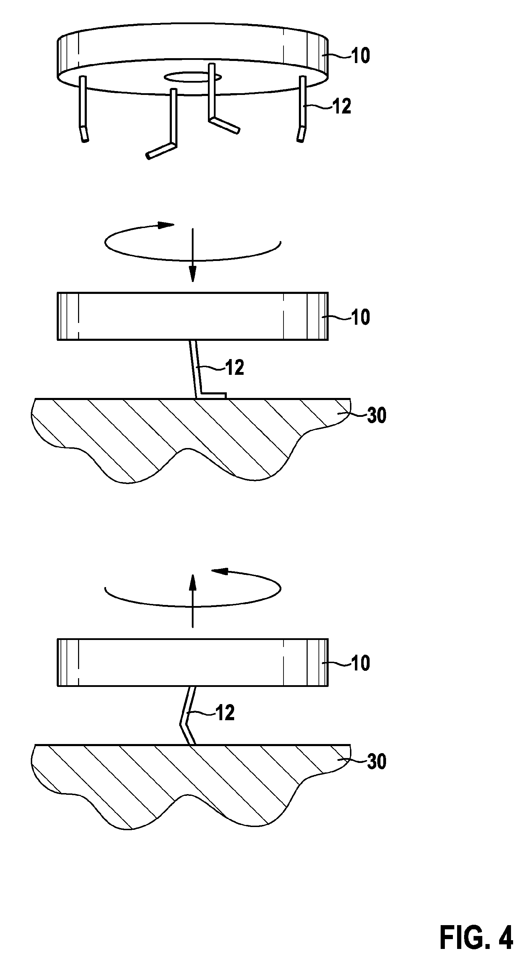

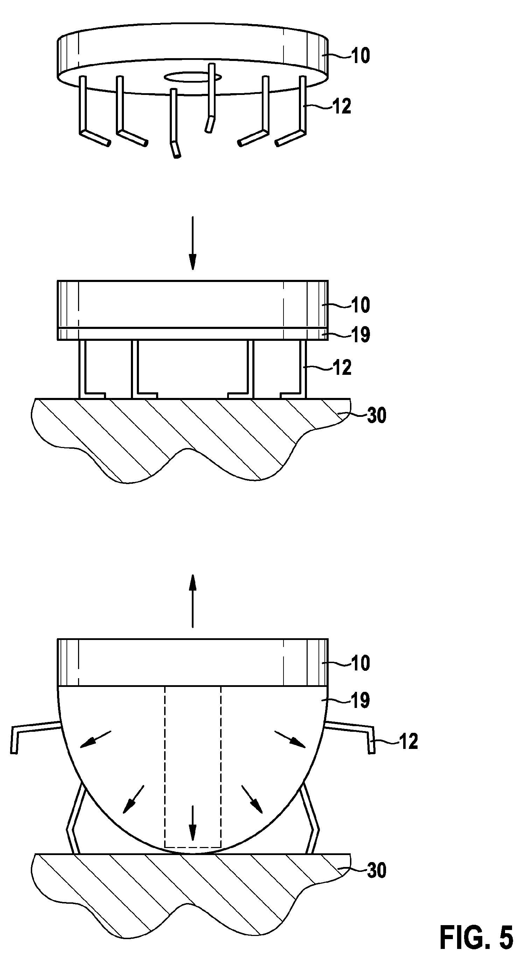

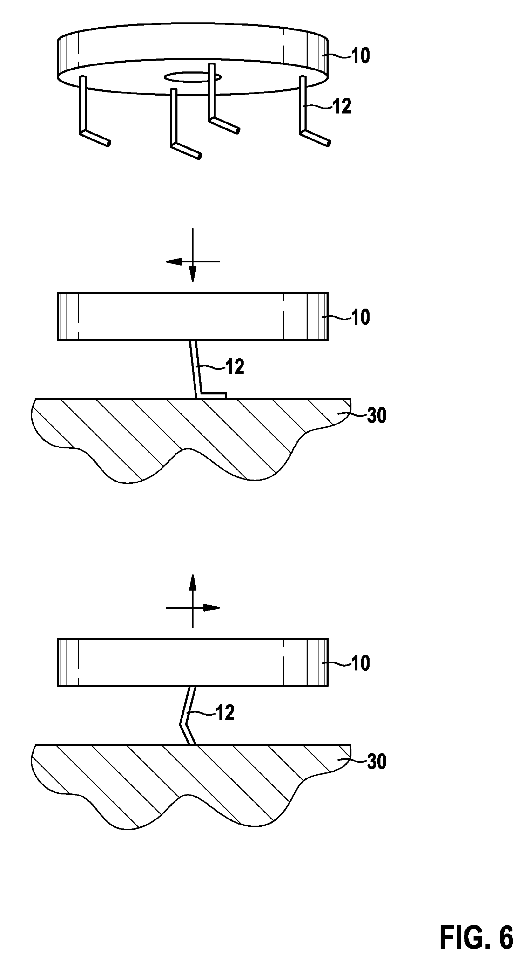

Auf den Figuren 4 bis 6 werden verschiedene Ausrichtungen der Stäbchen 12 illustriert, durch die eine Komponente durch eine definierte Bewegung vom Gewebe 30 wieder abgelöst werden kann. Eine schematische Darstellung einer Vorzugsbiegerichtung der Stäbchen 12 der haftvermittelnden Oberfläche 10, die ein Fixieren bei Drehung mit dem Uhrzeigersinn beziehungsweise Lösen bei Drehung gegen den Uhrzeigersinn ermöglicht, ist Figur 4 zu entnehmen.Eine Vorzugsbiegerichtung der Stäbchen 12 der haftvermittelnden Oberfläche 10, kann alternativ auch so vorgegeben werden, dass ein Lösen durch orthogonal wirkende Kräfte ermöglicht wird (Figur 5). Dazu können die Stäbchen 12 auf einer elastischen Membran 19 angeordnet werden, auf deren Rückseite zum Beispiel mit einem Stößel oder durch einen Eintrag eines Mediums zum Lösen Druck ausgeübt wird. Durch entsprechende Vorgabe der Vorzugsbiegerichtung der Stäbchen 12 der haftvermittelnden Oberfläche 10 kann auch ein Verschieben in eine Richtung ermöglicht werden (Figur 6).

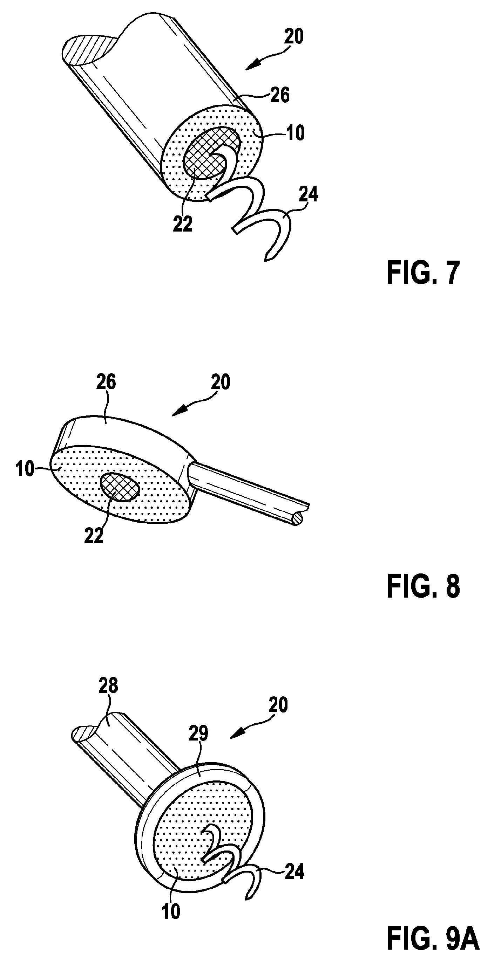

Figur 7 zeigt eine Spitze einer Herzelektrode 20 eines herkömmlichen Schrittmachers oder auch kabellosen Schrittmachers. Neben einer elektrisch aktiven Oberfläche 22 weist ein Kopf 26 der Elektrode 20 eine mittig auf der Stirnseite angeordnete Fixierschraube 24 zur mechanischen Verankerung im Epikard auf. Die haftvermittelnde Oberflächenstruktur 10 befindet sich um die Fixierschraube 24 herum und ist so ausgelegt, dass sie ein selbständiges Drehen des Körpers verhindert (siehe dazu Ausgestaltung gemäß Figur 4).

Figur 8 zeigt die Spitze einer epikardialen Herzelektrode 20. Die haftvermittelnde Oberfläche 10 ist erneut um die elektrisch aktive Oberfläche 22 auf dem Elektrodenkopf 26 angeordnet. Die Elektrode 20 wird einfach von außen auf das Herz gedrückt und fixiert selbständig durch die Fixationsfläche, ohne das Gewebe zu schädigen.

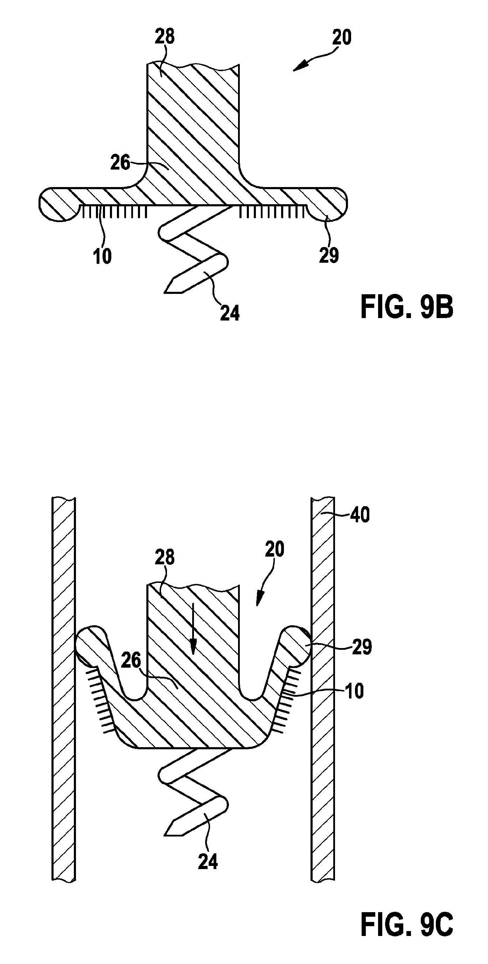

Die Figuren 9A bis 9C zeigen eine weitere Ausführungsform einer Herzelektrode 20 mit Fixierschraube 24 und haftvermittelnder Oberfläche 10 in drei verschiedenen Ansichten. Der Elektrodenkopf 26 weitet sich ausgehend von einem distalen Ende einer Elektrodenleitung 28 tellerförmig auf. Der tellerförmig ausgeweitete Bereich des Elektrodenkopfes 28 kann in Richtung der Elektrodenleitung 28 reversibel umgeklappt werden und besteht dazu aus einem elastischen Material. Der äußere Rand der Stirnseite des Elektrodenkopfes 26 ist verstärkt und agiert als Abstandshalter 29, wenn sich der umgeklappte Elektrodenkopf 26 in einem Einführbesteck 40 befindet (siehe Figur 9C).

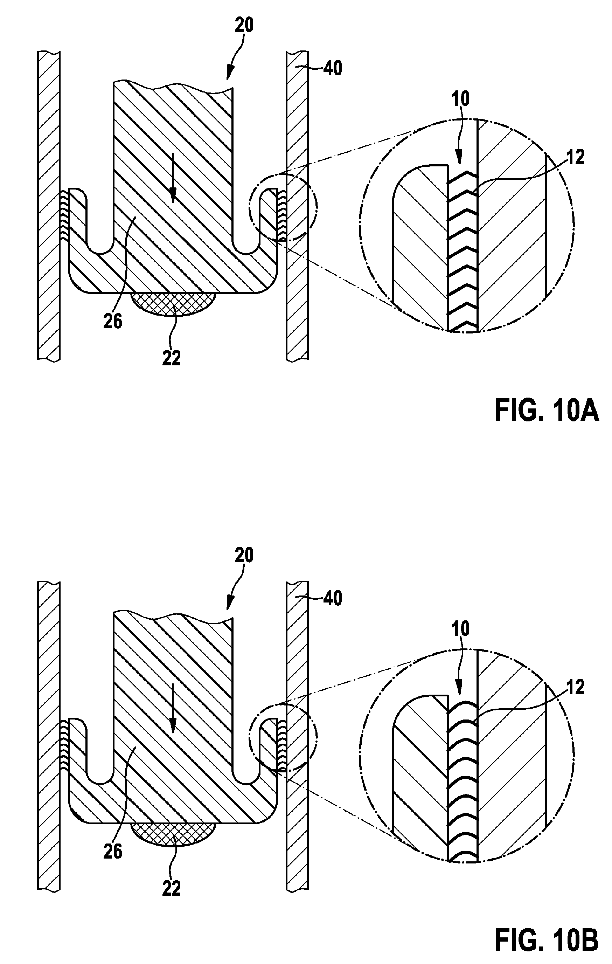

Den Figuren 10A und 10B sind zwei weitere Ausführungsformen von Herzelektroden 20 mit einer haftvermittelnden Oberfläche 10 zu entnehmen. Die Elektrode 20 weist einen mittig angeordneten elektrisch aktiven Bereich 22 auf, der auf einem tellerförmig erweiterten Elektrodenkopf 26 sitzt. Durch gezielte Ausrichtung der die haftvermittelnde Oberflächenstruktur 10 bildenden Stäbchen 12 ist ein Verschieben des Kopfes 26 im Einführbesteck 40 in eine Richtung möglich. Die haftvermittelnde Oberflächenstruktur 10 ist also so ausgelegt, dass eine vom Mittelpunkt des Elektrodenkopfes 26 radial nach außen wirkende Kraft einer Adhäsion der Oberflächenstruktur 10 an einer anliegenden Fläche entgegenwirkt.

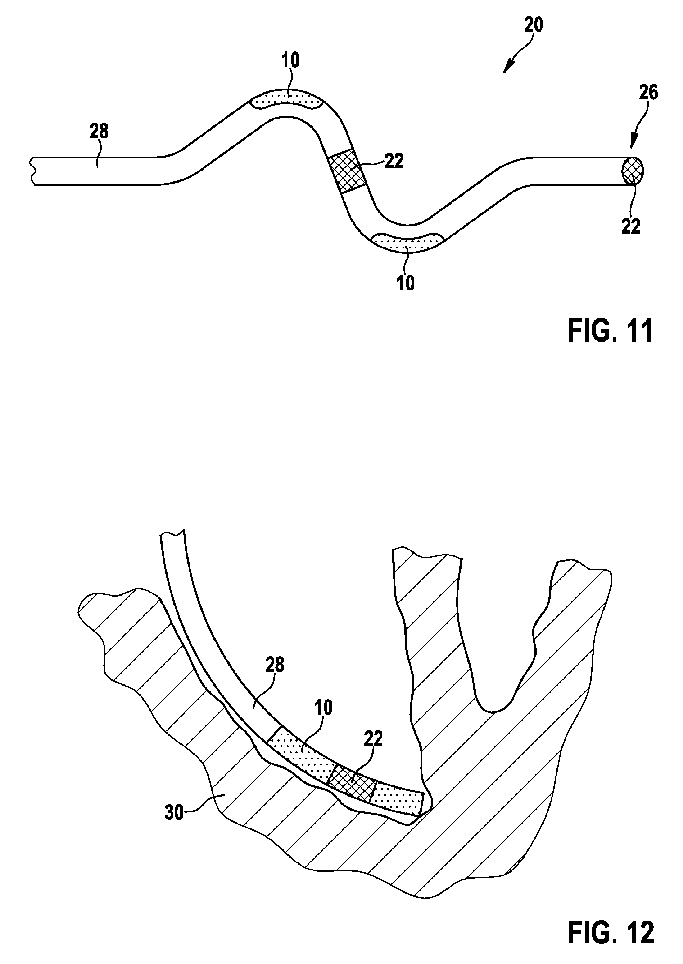

Figur 11 zeigt eine Elektrode 20 mit einer langgestreckten Elektrodenleitung 28 und einem distal an dieser angeordneten Elektrodenkopf 26. Die haftvermittelnde Oberflächenstruktur 10 ist hier in zwei verschiedenen Abschnitten der Elektrodenleitung 26 vorhanden. Konkret befinden sich die haftvermittelnden Oberflächenstrukturen 10 im Bereich einer Vorformung der Elektrodenleitung 28, die zur Abstützung an einer Gefäßwand dient. Die Positionierung von auf spezielle Weise gekrümmten und/oder verzweigten Elektrodenleitungen 28 wird also dadurch unterstützt, dass die vorgeprägte Grundform in vorgegebenen Bereichen eine haftvermittelnde Oberflächenstruktur 10 aufweist. Hierdurch ist ein besonders zuverlässiges Anliegen an der Herzwand gewährleistet.

Figur 12 ist eine weitere Ausführungsform der Elektrodenleitung 28 mit einer haftvermittelnden Oberfläche 10 zu entnehmen. Die dargestellte intrakardiale Herzelektrode 20 weist radial angeordnete Fixationsareale auf. Diese Darstellung zeigt eine aktiv fixierbare Elektrode 20, es ist auch eine passiv fixierbare atraumatische Ausführung denkbar

## Patentansprüche

1. Implantierbare Elektrode (20), **dadurch gekennzeichnet, dass** die Elektrode (20) einen Elektrodenkopf (26) mit einer haftvermittelnden Oberflächenstruktur (10) umfasst.

2. Elektrode nach Anspruch 1, bei der der Elektrodenkopf (26) sich ausgehend von einem distalen Ende einer Elektrodenleitung (28) tellerförmig aufweitet und der tellerförmig ausgeweitete Bereich des Elektrodenkopfes (26) in Richtung der Elektrodenleitung (28) reversibel umklappbar ausgelegt ist.

3. Elektrode nach Anspruch 2, bei der auf der Stirnseite des Elektrodenkopfes (26) Abstandshalter (29) angeordnet sind, die die haftvermittelnde Oberflächenstruktur (10) überragen.

4. Elektrode nach Anspruch 3, bei der die haftvermittelnde Oberflächenstruktur (10) so ausgelegt ist, dass eine vom Mittelpunkt des Elektrodenkopfes (26) radial nach außen wirkende Kraft einer Adhäsion der Oberflächenstruktur (10) an einer anliegenden Fläche entgegenwirkt.

5. Elektrode nach Anspruch 2, bei der der Elektrodenkopf (26) mittig eine Fixierschraube (24) aufweist und die haftvermittelnde Oberflächenstruktur (10) so ausgelegt ist, dass eine mit der Gewinderichtung der Fixierschraube (24) wirkende Kraft einer Adhäsion der Oberflächenstruktur (10) an einer anliegenden Fläche entgegenwirkt.

6. Elektrode nach einem der vorhergehenden Ansprüche, bei der die haftvermittelnde Oberflächenstruktur (10) aus einem polymeren Werkstoff geformt ist.

7. Elektrode nach Anspruch 6, bei der der polymere Werkstoff ein Silikon ist.

8. Elektrode nach einem der vorhergehenden Ansprüche, bei der die haftvermittelnde Oberflächenstruktur (10) eine Geckostruktur ist.

9. Implantierbare Elektrode (20) mit einer langgestreckten Elektrodenleitung (28) und einem distal an dieser angeordneten Elektrodenkopf (26), **dadurch gekennzeichnet, dass** die Elektrodenleitung (28) eine haftvermittelnde Oberflächenstruktur (10) aufweist.

10. Elektrode nach Anspruch 9, bei der die haftvermittelnde Oberflächenstruktur (10) im Bereich einer Vorformung der Elektrodenleitung (28), die zur Abstützung an einer Gefäßwand dient, angeordnet ist.

11. Elektrode nach einem der Ansprüche 9 oder 10, bei der die haftvermittelnde Oberflächenstruktur (10) aus einem polymeren Werkstoff geformt ist.

12. Elektrode nach Anspruch 11, bei der der polymere Werkstoff ein Silikon ist.

13. Elektrode nach einem der Ansprüche 9 bis 12, bei der die haftvermittelnde Oberflächenstruktur (20) eine Geckostruktur ist.
